# EUROPEAN PATENT APPLICATION

(11) **EP 2 236 881 A1**
(43) Date of publication of application: **06.10.2010**
(21) Application number: 08865996.6
(22) Date of filing: 28.11.2008
(51) Int. Cl.: F16K 31/06, A61B 5/0225

(54) **SOLENOID VALVE**

(30) Priority: 28.12.2007 JP 2007340143; 28.12.2007 JP 2007340144
(71) Applicant: Hosiden Corporation, Yao-shi, Osaka 581-0071 (JP); Terumo Kabushiki Kaisha, Shibuya-ku Tokyo 151-0072 (JP)
(72) Inventor: KATAOKA, Hideyuki, Kashiwara-shi Osaka 582-0027 (JP); KOUSA, Shinichiro, Kashiwara-shi Osaka 582-0027 (JP); ASAMA, Kouichirou, Ashigarakami-gun Kanagawa 259-0151 (JP); OZAWA, Hitoshi, Fujinomiya-shi Shizuoka 418-0015 (JP); SASAGAWA, Yuuki, Fujinomiya-shi Shizuoka 418-0015 (JP); HAGI, Kouji, Ashigarakami-gun Kanagawa 259-0151 (JP)
(74) Representative: Lemcke, Brommer & Partner
(86) International application number: PCT/JP2008/071642
(87) International publication number: WO 2009/084353

(57) **Abstract**

There is provided a solenoid valve that does not require high precision working technique for its movable member and that is light-weighted and provides high operation stability. The solenoid valve includes a fixed iron core (12), a solenoid coil (13), a nozzle unit (3) having a passageway (3a), a valve body (6) for closing the passageway (3a), and a movable member movable, in accordance with energization state to the solenoid coil (13), between a first posture where the valve body (6) closes the passageway (3a) and a second posture where the passageway (3a) is opened. The movable member comprises a pivotal member (5) pivotably operated between the first posture and the second posture. At one end of the pivotal member (5), there is provided an attracted portion (5a) which is electro-magnetically attracted to the fixed iron core (12) thereby to realize the first posture. At the other end of the pivotal member (5), there is provided the valve body (6) for closing the passageway (3a).

## Description

### Technical Field

The present invention relates to a solenoid valve including a fixed iron core extending along an axis, a solenoid coil disposed around the fixed iron core, a nozzle unit having a passageway, a valve body for closing the passageway, and a movable member supporting the valve body and movable, depending on an energization state to the solenoid coil, between a first posture for closing the passageway with the valve body and a second posture for opening the passageway.

### Background Art

As prior-art document information relating to this type of solenoid valve, there is Patent Document 1 identified below. With the solenoid valve disclosed in this Patent Document 1, the movable member is provided in the form of a movable iron core which is slidable along an inner face of a cylindrical bobbin of the solenoid coil. And, at one end of this movable iron core, there is mounted the valve body for closing the passageway. The nozzle unit is composed by a fixed iron core. Between the movable iron core and the nozzle unit, there is disposed a coil spring for urging the movable iron core toward the second posture. In accordance with the energization state to the solenoid coil, both the movable iron core and the fixed iron core are magnetized, whereby there is formed a magnetic flux extending continuously past the outer side of the coil from the movable iron core to the fixed iron core (nozzle unit). As the movable iron core is magnetically attracted to the first posture adjacent the nozzle unit as the fixed iron core so as to render this magnetic flux as short as possible, the passageway is closed with the valve body approaching the nozzle unit. Upon release of the energization to the solenoid coil, the movable iron core is returned to the second posture under the urging force of the coil spring, whereby the passageway is opened.

However, with the solenoid valve disclosed in Patent Document 1, high precision working technique was needed for the movable iron core (movable member) and for the bobbin in order to allow the movable iron core to be slidably supported to the inner face of the bobbin without looseness and with smoothness. Further, since the nozzle unit acts also as the fixed iron core for electromagnetically attracting the movable iron core, this unit was necessarily formed of a magnetic material, thus resulting in significant mass. Moreover, it was needed to dispose this nozzle unit acting also as the fixed iron core coaxially with the movable member. Furthermore, if a portion of the coil spring was clamped between the movable iron core and the nozzle unit, this would prevent the movable iron core from being disposed in sufficient vicinity to the nozzle unit. Hence, there was the possibility of the valve body not providing reliable closure.

In view of the various drawbacks of the solenoid valves of the conventional art shown above, the primary object of the present invention is to provide a solenoid valve that does not require high precision working technique for the movable member.
A further object of the invention is to provide a solenoid valve that is as light-weighted and compact as possible.
A still further object of the invention is to provide a solenoid valve that does not impose significant restriction on the disposing position of its nozzle unit.
A still further object of the invention is to provide a solenoid valve that is stable in its operation.
Patent Document 1: Japanese Unexamined Patent Application Publication No. 2006-29362 (paragraphs 0014, 0016, Figs. 1 and 2).

### Disclosure of the Invention

A solenoid valve, according to the present invention, comprises:
a fixed iron core extending along an axis;
a solenoid coil disposed around the fixed iron core;
a nozzle unit having a discharge passageway;
a valve body for closing the discharge passageway of the nozzle unit; and
a movable member supporting the valve body and movable, depending on an energization state to the solenoid coil, between a first posture for closing the discharge passageway with the valve body and a second posture for opening the discharge passageway;
   **characterized in that**
the movable member comprises a pivotal member supported to be pivotable via a pivot axis between the first posture and the second posture;
at one end of the pivotal member, there is provided an attracted portion configured to realize the first posture as this attracted portion is electromagnetically attracted to the fixed iron core in accordance with energization to the solenoid coil; and
at the other end away from said one end across the pivot axis, there is provided the valve body for closing the discharge passageway in said first posture.

Therefore, with the solenoid valve according to the present invention, the movable member can be provided as a pivotal member that is pivoted between a first posture and a second posture. There is no need to provide it in the form of a movable iron core slidably displaced along the inner face of the bobbin. As a result, it has become possible to eliminate the need for the high precision work for the movable iron core (movable member) and the inner face of the bobbin, whereby the manufacture costs have been restricted. Further, since the pivotal member is configured to assume the first posture based on the attraction of the attracted portion by the fixed iron core, there is no need to form the nozzle unit as a magnetic body having large mass. Hence, a light-weight solenoid valve has been obtained. Moreover, thanks to no need to dispose the nozzle unit coaxially with the movable member, a greater degree of design freedom has been obtained.

According to a further characterizing feature of the present invention, there is provided an urging mechanism for urging the pivotal member toward the second posture where the valve body is removed from an opening of the discharge passageway.
With this construction, when the pivotal member is switched over to the second posture, the valve body will be removed from the opening of the discharge passageway reliably and speedily by the urging mechanism. Therefore, discharge of fluid can be effected speedily and reliably, upon release of energization to the solenoid coil.

Therefore, the inventive solenoid valve can be used as a discharge valve employed in a medical apparatus, e.g. an automated sphygmomanometer, for discharging air at a constant and very low speed. In particular, the invention may be employed in a finger sphygmomanometer, a wrist sphygmomanometer, an ear sphygmomanometer, having a small cuff capacity. Further, the invention may be suitably employed in a brachial sphygmomanometer (an integrated sphygmomanometer integrating a cuff and a body). Furthermore, the inventive valve can be suitably employed in any other medical apparatuses such as an air discharge valve in a massaging apparatus for ankles, a respiration synchronizer (demand valve) in an oxygen concentrator, an air discharge valve in an air cylinder forming a clamp mechanism of an automated peritoneal dialysis apparatus, etc.

According to a still further characterizing feature of the present invention, the valve body comprises an elastic member having a closing portion that is elastically pressed against the opening of the nozzle unit under the first posture; and
the urging mechanism includes an operation assisting portion provided in a housing which houses the pivotal member therein and a discharge operation portion formed as a projecting portion of the valve body so as to elastically urge the pivotal member toward the second posture in response to engagement thereof with the operation assisting portion.

With the above construction, the valve body integrally including the closing portion for closing nozzle unit and the discharge operation portion for switching the pivotal member to the second posture in cooperation with a portion (the operation assisting portion) of the housing can be produced in a single step of e.g. injection molding of elastomer. As a result, the manufacture costs reduction and the reduction of the number of parts can be realized.

According to a still further characterizing feature of the present invention, the discharge operation portion is configured to maintain its engagement with the operation assisting portion under both the first posture and the second posture.
With this construction, there is no risk of the discharge operation portion being inadvertently switched over to the first posture without being engaged with the operation assisting portion. Consequently, the discharge operation portion achieves its intended function in cooperation with the operation assisting portion in a reliable manner.

According to a still further characterizing feature of the present invention, there is provided a restricting means for restricting movement of a pivot axis included in the pivotal member.

With the above construction, due to the provision of the restricting member, movement of the pivot axis of the pivotal member due to pressure of the discharged fluid is restricted. As a result, even when the fluid is discharged rapidly from the nozzle unit during the switchover of the movable member to the second posture, there will not occur moving about of the valve body by this discharge pressure, so that the fluid discharge can proceed in a stable manner.

Therefore, the inventive solenoid valve can be used as a discharge valve employed in a medical apparatus, e.g. an automated sphygmomanometer, for discharging air at a constant and very low speed. In particular, the invention may be employed in a finger sphygmomanometer, a wrist sphygmomanometer, an ear sphygmomanometer, having a small cuff capacity. Further, the invention may be suitably employed in a brachial sphygmomanometer (an integrated sphygmomanometer integrating a cuff and a body). Furthermore, the inventive valve can be suitably employed in any other medical apparatuses such as an air discharge valve in a massaging apparatus for ankles, a respiration synchronizer (demand valve) in an oxygen concentrator, an air discharge valve in an air cylinder forming a clamp mechanism of an automated peritoneal dialysis apparatus, etc.

According to a still further characterizing feature of the invention, the restricting means includes a pair of retaining grooves disposed in opposition to each other in front and back faces of the pivotal member and a plate-like member having a slit-like cutout that comes into engagement with the pair of retaining grooves simultaneously.

With this construction, simply by inserting the slit-like cutout portion of this plate-like member into the pair of retaining grooves, there can be obtained the arrangement needed for supporting the pivotal member without movement of the pivot axis of this pivotal member.

According to a still further characterizing feature of the present invention, a plate-like yoke is disposed adjacent the solenoid coil; and
the restricting means includes a first retaining hole formed in the pivotal member to extend along the pivot axis, a second retaining hole formed at the leading end of the yoke, and a shaft member inserted into the first retaining hole and the second retaining hole simultaneously.

With the above construction, because of the use of the support arrangement with the shaft member inserted into the retaining holes, the movement of the pivot axis of the pivotal member can be restricted even more reliably. Further, since the pivotal member is supported to the yoke which is a constituting element of the solenoid, without providing any special member for pivotally supporting the pivotal member, the number of parts can be further reduced.

### Brief Description of the Drawings

[Fig. 1] is a broken side view showing a solenoid valve according to the present invention,
[Fig. 2] is a broken plane view of the solenoid shown in Fig. 1,
[Fig. 3] is an exploded perspective view of the solenoid valve shown in Fig. 1,
[Fig. 4] is a perspective view showing principal portions of an automated sphygmomanometer,
[Fig. 5] is a schematic block diagram showing the construction of the automated sphygmomanometer,
[Fig. 6] is a schematic block diagram showing the construction of an oxygen concentrator,
[Fig. 7] is a broken side view of principal portions showing a solenoid valve according to a further embodiment,
[Fig. 8] is a broken side view of principal portions showing a solenoid valve according to a still further embodiment,
[Fig. 9] is a broken plane view of the solenoid valve shown in Fig. 8,
[Fig. 10] is an exploded perspective view of the solenoid valve shown in Fig. 8,
[Fig. 11] is a broken side view showing principal portions showing a modified embodiment of the solenoid valve shown in Fig. 8,
[Fig. 12] is a broken plane view of the solenoid valve shown in Fig. 11, and
[Fig. 13] is an exploded perspective view of the solenoid valve shown in Fig. 11.

### Best Modes of Embodying the Invention

Next, best modes of embodiments of the invention will be described with reference to the accompanying drawings. It is understood however that the present invention is not limited to these embodiments.
In the following, the solenoid valve relating to the present invention will be explained by way of an exemplary case where the inventive solenoid valve is employed as an apparatus constituting a portion of an automated sphygmomanometer having a cuff (tourniquet) to be wound about a tested portion such as a patient's arm. In this case, the solenoid valve provides the function of discharging at one time to the outside an amount of compressed air which was fed into the cuff with a pump, after completion of blood pressure determination.
It is understood, however, that the inventive solenoid valve is not limited to the use in a sphygmomanometer, but can be applied to a wide variety of instruments or apparatuses, in particular, medical instruments or apparatuses, that require fluid rate control.

### [First Embodiment]

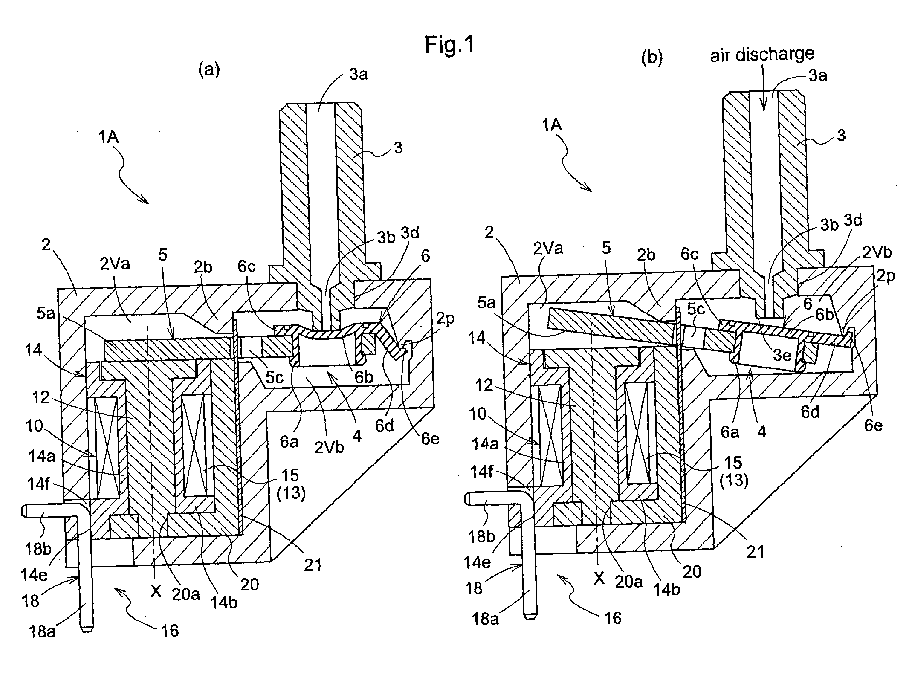

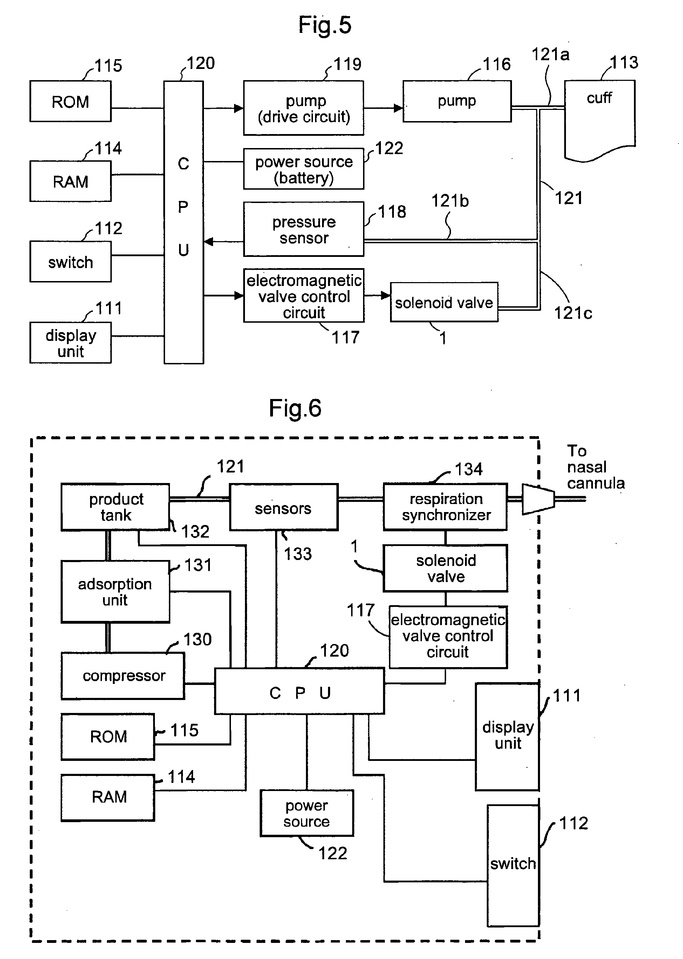

A solenoid valve 1A shown in Figs. 1-3 includes a housing 2 made of resin. The housing 2 includes an inner cavity having a generally L-shaped cross section, with only one lateral side thereof being open. This cavity includes a first cavity portion 2Va extending vertically in Fig. 1 and a second cavity portion 2Vb extending laterally from the vicinity of the upper end of the first cavity portion 2Va. To the outer side of the housing 2, there is mounted a nozzle unit 3 having a passageway 3a for air discharge (air exhaust). The base end of the passageway 3a is communicated to the second cavity portion 2Vb. The second cavity portion 2Vb incorporates therein a valve body 6 for closing the passageway 3a. The first cavity portion 2Va incorporates therein a solenoid unit 10 for switching over between a state where the valve body 6 closes the passageway 3a and a state where the valve body 6 opens it. The nozzle unit 3 is pressure-inserted and fixed within a through hole 2H formed in the upper face of the housing 2. To the nozzle unit 3, there is externally and air-tightly engaged and connected an air exhaust pipe 121c (see Fig. 5) communicated to an air feed pipe 131 (to be described later) for feeding compressed air from the pump to the cuff.

The solenoid unit 10 includes a fixed iron core 12 extending along an axis X, a solenoid coil 13 for selectively magnetizing the fixed iron core 12, and a yoke 20 for converging a magnetic field generated by the solenoid coil 13. The solenoid coil 13 includes a bobbin 14 disposed outwardly around the outer peripheral face of the fixed iron core 12, a wire 15 wound in the form of a coil about a cylindrical portion 14a of the bobbin 14, and an energizing portion 16 for energizing (power feeding to) the wire 15.

A valve portion 4 includes a pivotal member 5 (movable member) supported to an upper end face of plate-like member 21 disposed erect laterally of the solenoid coil 13. The pivotal member 5 is supported to be pivotable via a pivot axis Y formed within the plane of the plate-like member 21. At one end of the pivotal member 5, there is provided an attracted portion 5a which is attracted to an upper end of the fixed iron core 12 that is magnetized in response to energization to the solenoid coil 13. As the attracted portion 5a is attracted to the upper end of the fixed iron core 12, the pivotal member 5 assumes a first posture for closing the passageway 3a of the nozzle unit 3, as shown in Fig. 1 (a).

Adjacent the other end of the pivotal member 5 which end corresponds to the opposite end away from the attracted portion 5a across the pivot axis, a circular through hole 5b is formed. Within this through hole 5b, the valve body 6 formed of an elastic material such as rubber is retained. The elastic material forming the valve body 6 can be e.g. a synthetic rubber (e.g. NBR) having a shore hardness value of about 40. However, the material is not limited thereto, but any other material having appropriate elasticity for use as a valve body in the invention can be used also. The valve body 6 integrally includes a cylindrical retained portion 6a which is engaged and inserted from above the through hole 5b of the pivotal member 5 , a flat disc-like upper face portion 6b (an example of "closing portion") that closes the upper end of the retained portion 6a, a flange portion 6c extending radially outward from the circumference of the upper end of the retained portion 6a, and an extension portion 6d (an example of "discharge operation portion") extending further radially to one side from a portion of the flange portion 6c.

Under the first posture where the attracted portion 5a is attracted to the upper end of the fixed iron core 12, as shown in Fig. 1 (a), the upper face portion 6b of the valve body 6 is pressed against an open end face 3e of the nozzle unit 3, whereby the passageway 3a of the nozzle unit 3 is closed. In this, the extension portion 6d of the valve body 6 is elastically deformed to be bent downward, by a projection-like, operation assisting portion 2p formed downward from the inner face of the housing 2. At the same time, the upper face portion 6b of the valve body 6 is also elastically deformed, with its center portion projecting downward. Incidentally, inside the passageway 3a of the nozzle unit 3, there is formed an orifice with an inner diameter of about 0.3 mm which is very small relative to the inlet opening formed at the upper end of the passageway 3a. As this orifice 3b extends continuously to the lower end of the passageway 3a against which the valve body 6 is pressed, reliable closure is made possible even with a relatively small pressing force for pressing the valve body 6 against the nozzle unit 3.

Upon release of energization (power supply) to the solenoid coil 13, this eliminates the magnetization of the fixed iron core 12 and the magnetic attraction of the attracted portion 5a. Hence, the pivotal member 5 is switched over to a second posture shown in Fig. 1 (b), due to elastic resilience of the extension portion 6d of the valve body 6 that urges this portion 6d to return to its shape extending straight along the upper face portion 6b, elastic resilience of the upper face portion 6b of the valve body 6 which urges this face portion 6b to return to its flat shape without any projection and also the pressure of air discharged from the cuff (in case compressed air is present in the cuff of the sphygmomanometer). Under this second posture, the condition of the valve body 6 being pressed against the open end portion 3c is released, so the passageway 3a is opened.

The extension portion 6d of the valve body 6 has a rectangular shape in its plane view and from the vicinity of its straight side at the leading end, there is upwardly extended a pawl-like projection 6e engageable with a sharp end of the operation assisting portion 2p. This pawl-like portion 6e, which has a triangular cross section and extends straight through the plane of Fig. 1, provides a function of causing the extension portion 6e to engage the operation assisting portion 2p in a reliable manner when the pivotal member 5 is under the first posture. Further, since the pawl-like projection 6e is configured to maintain the engaged posture engaged with the operation assisting portion 3p under the second posture already, there is no risk of being switched over to the first posture without engagement between the pawl-like projection 6e and the operation assisting portion 2p. The pawl-like projection 6e can be sized to have a height of 0.2 to 0.4 mm approximately. The value is not limited thereto, however.

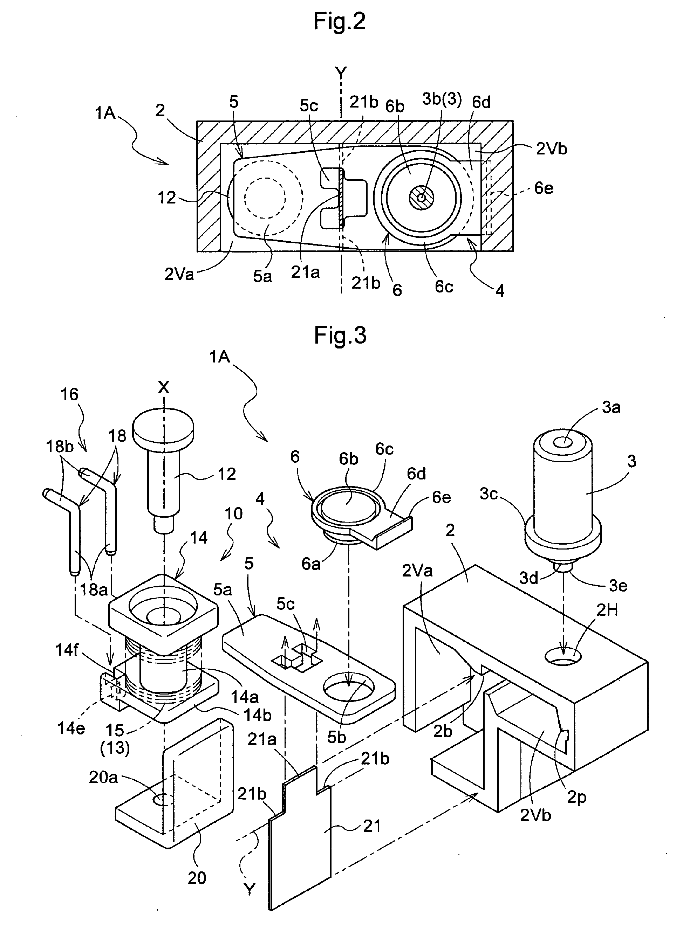

As shown in Fig. 1 and Fig. 3, the plate-like member 21 supporting the pivotal member 5 is clamped and fixed between the yoke 20 and the housing 2 to extend substantially parallel to the axis X. At the center of the upper end of the plate-like member 21, there is provided in the form of a step, a projecting portion 21a projecting upward. At the center of the pivotal member 5, there is formed a slit 5c having a length corresponding to the width of the projecting portion 21a. The pivotal member 5 is supported on a pair of shoulders 21b extending straight to the right and left of the projecting portion 21a, with this projecting portion 21a being inserted in the slit 5c. From an inner face of the cavity portion 2Va of the housing 2, a retaining projection 2b having a wedge-like cross section extends downward. The retaining projection 2b constitutes an anti-withdrawal portion for preventing inadvertent withdrawal of the projecting portion 21a from the slit 5c, regardless of the posture of the solenoid valve 1A, e.g. even when the solenoid valve 1A is used under vertically reversed posture relative to the posture shown in Fig. 1.

The energizing portion 16 includes a pair of bar-like terminals 18 bent in the L-shape. Each bar-like terminal 18 is retained as being pressed into and engaged within a groove formed in a lower flange portion 14b projecting radially from the lower end of the bobbin 14. More particularly, the terminal 18 consists of a straight extending terminal body 18a and a connected portion 18b extending integrally at a right angle from the upper end of the terminal body 18a.
The lower flange portion 14b of the bobbin 14 defines a receiving hole 14e capable of receiving therein the terminal body 18a along the axis X and an engaging groove 14f communicated to the receiving hole 14e and extending radially. In operation, the terminal body 18a of the terminal 18 will be inserted from above the receiving hole 14e of the lower flange portion 14b and then the connected portion 18b of the terminal body 18 will be pressed into the engaging groove 14f. With these simple and easy operations, fixing of the terminal 18 to the bobbin 14 will be completed. The wire 15 constituting the solenoid coil 13 is directly welded to the leading end portion or intermediate portion of the connected portion 18b. With the engagement between the engaging groove 14f and the connected portion 18b, inadvertent withdrawal of the terminal 18 along the axis X and the pivotal displacement of the terminal 18 within the receiving hole 14e are restricted.

Next, an example of assembly operation of the solenoid valve 1A will be described next.
(1) The wire 15 is wound around the cylindrical portion 14a of the bobbin 14, thus forming the solenoid coil 13.
(2) The terminal bodies 18a of the terminals 18 are inserted from above into the receiving hole 14e of the lower flange portion 14b and the connected portions 18b of the terminals 18 are pressed into the engaging grooves 14f, whereby the pair of terminals 18 are fixed to the bobbin 14. The opposed terminal ends of the wire 15 are welded to the connected portions 18b.
(3) The fixed iron core 12 is inserted into the cylindrical portion 14a of the bobbin 14 to which the solenoid coil 13 is supported. After the lower end of the fixed iron core 12 is inserted into the through hole 20a of the yoke 20, this is fastened by caulking.
(4) The retained portion 6a of the valve body 6 is fitted into the through hole 5b of the pivotal member 5.
(5) The pivotal member 5 is placed on the upper end of the plate-like member 21. In this, the projecting portion 21a of the plate-like member 21 is inserted into the slit 5c of the pivotal member 5, so that the lower face of the pivotal member 5 may be supported on the shoulders 1b provided on the right and left sides of the projecting portion 21a of the plate-like member 21.
(6) The assembly consisting of the pivotal member 5 and the plate-like member 21 is accommodated within the cavity portion 2Va of the housing 2.
(7) The assembly of the solenoid coil 13 is press-fitted laterally into the cavity portion 2Va of the housing 2. In doing this, care is taken such that the plate-like member 21 accommodated there in advance may be clamped and fixed between the yoke 20 and the inner wall of the housing 2.
(8) The nozzle unit 3 is attached to the housing 2. Adjacent the lower end of the nozzle unit 3, there are formed a flange 3c which is generally circular in its plane view and a small-diameter boss portion 3d extending downward from the flange 3c. The boss portion 3d is pres-fitted into a through hole 2H of the housing 2 so that the lower end face of the flange 3c may be placed in gapless contact with the upper face of the housing 2.

### (Application Examples)

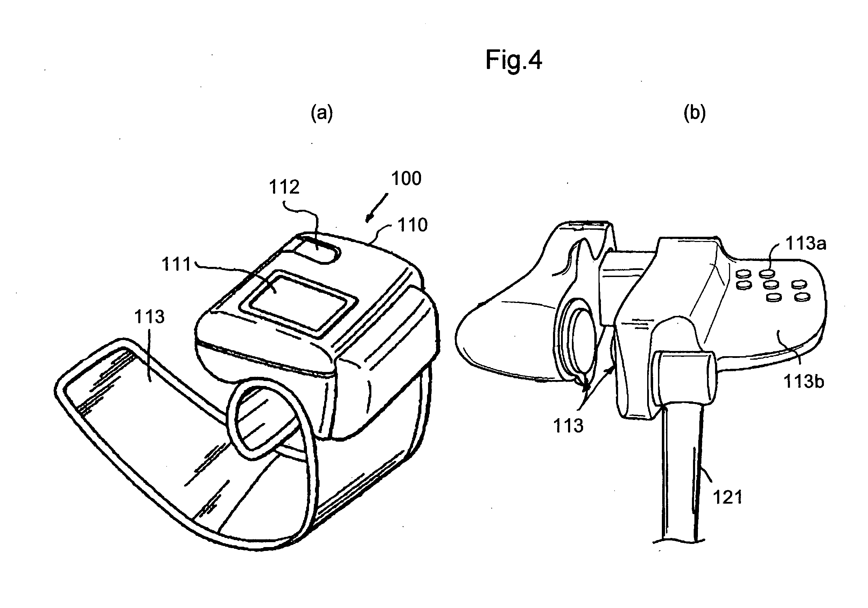

Examples of application of the solenoid valve 1A to medical apparatuses are shown in Fig. 4. Fig. 4 (a) shows an outer appearance of a wrist sphygmomanometer 100 as an example of an automated sphygmomanometer using the solenoid valve 1A. Fig. 4 (b) shows an outer appearance of a cuff device of an ear type sphygmomanometer. Further, Fig. 5 shows a schematic block diagram of an automated sphygmomanometer including the solenoid valve 1A.
As shown in Fig. 4 (a), the wrist sphygmomanometer 100 includes such components as a main body 110, a display unit 111 for displaying measurement values or the like, a switch 112 for power ON/OFF, a cuff 113, etc. Further, as shown in Fig. 4 (b), the cuff device of the ear type sphygmomanometer includes such components as an air feed pipe 121 made of an elastic tube (incorporating signal wires when needed), two cuffs 113 disposed in opposition to each other across an ear (preferably, antilobium) an anti-slip portion 113a, a handle 113b, etc.

As shown in Fig. 5, each one of these sphygmomanometers is comprised of a dry battery 122 as a power source unit, a pump 116 for feeding pressure air to the cuff (air bag) 113, the solenoid valve 1A of the invention for discharging air from the cuff 113, a pressure sensor 118 for detecting air pressure inside the cuff 113, an electromagnetic valve control circuit 117 for controlling drive of the solenoid valve 1A according to a measurement value of the pressure sensor 118, a CPU 120 as a controller for controlling the entire apparatus, a power switch 112 connected to the CPU 120, the display unit 111, and a ROM 115 for storing therein various programs to be executed by the CPU 120, a RAM 114 functioning as a work area for the program executed by the CPU 120 and functioning also as a storage means for temporarily storing setting input conditions, data or the like at the time of program processing, etc. Incidentally, the CPU 120 includes also a calculating means for calculating the maximum blood pressure (systolic arterial pressure), the minimal blood pressure (diastolic pressure), etc.

The air feed pipe 121 includes a main pipe 121a for feeding compressed air from the pump 116 to the cuff 113, a detection pipe 121b for establishing communication between the main pipe 121a and the pressure sensor 118, and an air discharge pipe 121c communicated to the main pipe 121a so as to discharge the high pressure air inside the cuff 113 to the outside by the solenoid valve 1A.
Further, a portable air massaging apparatus has a similar construction to that of the wrist sphygmomanometer described above. But, this apparatus stores a plurality of massaging patterns in its ROM 115, so that a desired one of the massage pattern can be selected therefrom with a selector switch.

Fig. 6 shows a schematic block diagram of an oxygen concentrator including the solenoid valve 1A, as a further example of application of the solenoid valve in a medical apparatus. This oxygen concentrator includes a power source (AC power and/or battery) 122 as a power unit, an adsorption unit 131 for selectively adsorbing nitrogen, a compressor 130 for feeding compressed air to the adsorption unit 131, a product tank 132 holding therein oxygen concentrated to 90% or more, a zirconia type or supersonic type sensor 133 for determining an oxygen concentration, an oxygen rate, etc., a respiration synchronizer 134 for feeding the concentrated oxygen to a nose cannula in synchronism with a patient's respiration, an electromagnetic valve control circuit 117 for controlling driving of the solenoid valve 1A, a CPU 120 for controlling the entire apparatus, the power switch 112 and a display unit 111 connected to the CPU 120, a ROM 115 for storing therein various programs to be executed by the CPU 120, a RAM 114 functioning as a work area for the program executed by the CPU 120 and functioning also as a storage means for temporarily storing setting input conditions, date or the like at the time of program processing, etc.

### [Second Embodiment]

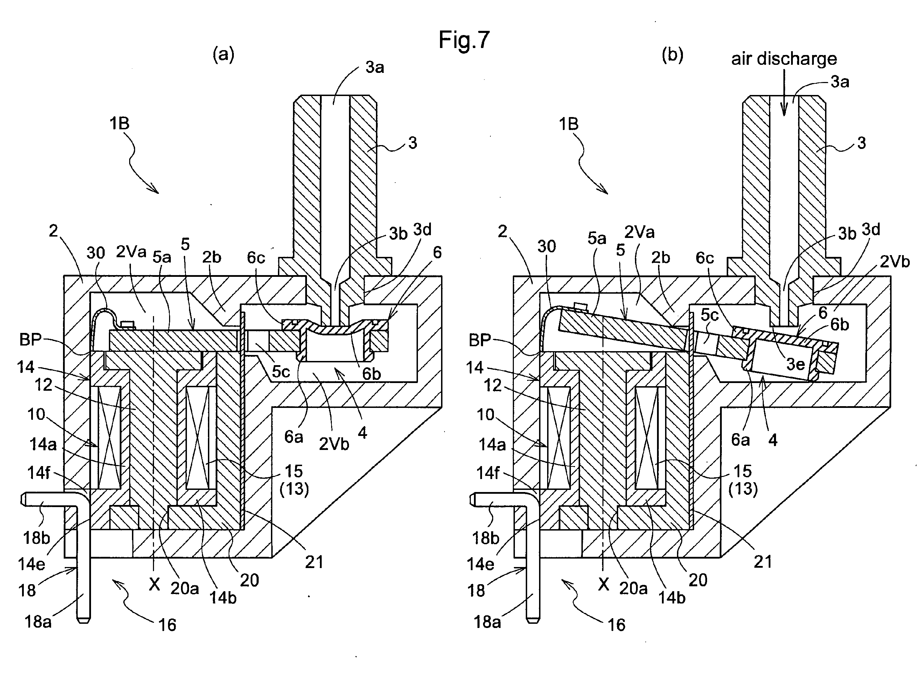

Fig. 7 shows a solenoid valve 1B according to a second embodiment wherein an urging mechanism for urging the pivotal member 5 toward the second posture is provided on the opposite side of the pivotal member away from valve body 6, that is, adjacent the end portion of the attracted portion 5a.
In this, the valve body 6 does not have the extension portion 6d and the urging mechanism comprises a discharge operation piece 30 (an example of "discharge operation portion") attached to the attracted portion 5a of the pivotal member 5. The discharge operation piece 30 is a plate-like member formed of elastomer attached so as to extend radially outward from the attracted portion 5a and its leading end is disposed in abutment against a border portion BP between the upper end face of the bobbin 14 and the inner face of the housing 2.

Under the first posture where the attracted portion 5a is magnetically attracted to the upper end of the fixed iron core 12 with energization to the solenoid coil 13, as shown in Fig. 7 (a), the valve body 6 closes the passageway 33a of the nozzle unit 3. In this condition, the discharge operation piece 30 is elastically deformed into a significantly stressed cross sectional shape, with a portion adjacent the center thereof being flexed with a small curvature of radius.
Next, upon release of energization to the solenoid coil 13, this substantially eliminates the magnetization of the fixed iron core 12 and the attraction of the attracted portion 5a, so that the pivotal member 5 will be switched over to the second posture shown in Fig. 7 (b), due to the elastic resilience of the discharge operation piece 30 tending to return to the less stressed shape generally flexed with a large curvature of radius toward the border portion BP, the elastic resilience of the upper face portion 6b of the valve body 6 tending to return to the flat shape without projection and the pressure of the air discharged from the cuff.

### [Third Embodiment]

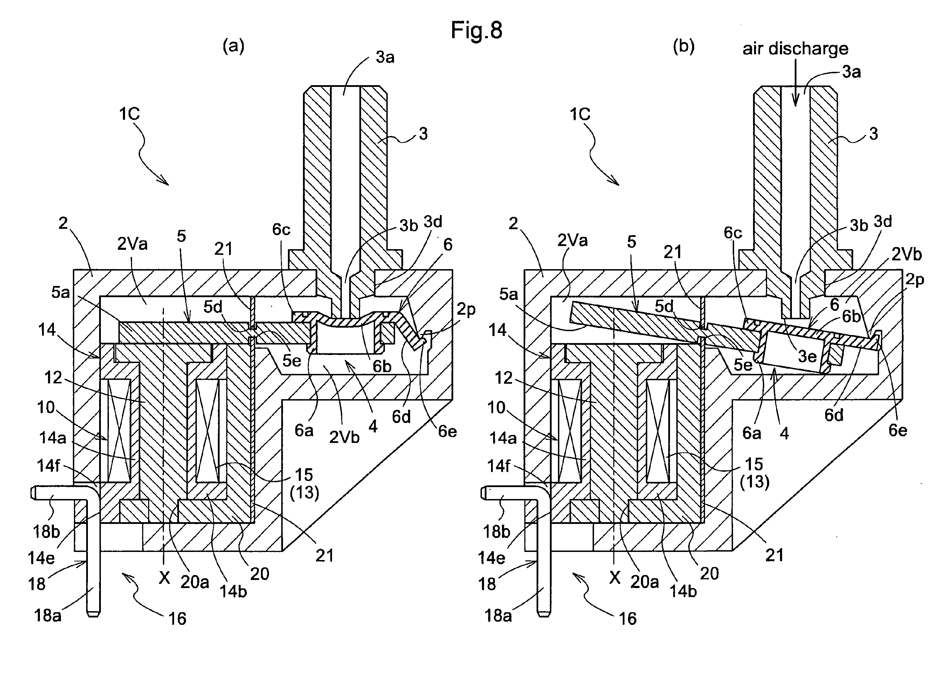

A solenoid valve 1C shown in Figs. 8-10 according to a third embodiment includes a housing 2 made of a resin. The housing 2 includes an inner cavity having a generally L-shaped cross section, with only one lateral side thereof being open. This cavity includes a first cavity portion 2Va extending vertically in Fig. 8 and a second cavity portion 2Vb extending laterally from the vicinity of the upper end of the first cavity portion 2Va. To the outer side of the housing 2, there is mounted a nozzle unit 3 having a passageway 3a for air discharge (air exhaust). The base end of the passageway 3a is communicated to the second cavity portion 2Vb. The second cavity portion 2Vb incorporates therein a valve body 6 for closing the passageway 3a. The first cavity portion 2Va incorporates therein a solenoid unit 10 for switching over between a state where the valve body 6 closes the passageway 3a and a state where the valve body 6 opens it. The nozzle unit 3 is pressure-inserted and fixed within a through hole 2H formed in the upper face of the housing 2. To the nozzle unit 3, there is externally and air-tightly engaged and connected an air exhaust pipe 121c (see Fig. 5) communicated to an air feed pipe 121 (to be described later) for feeding compressed air from the pump to the cuff.

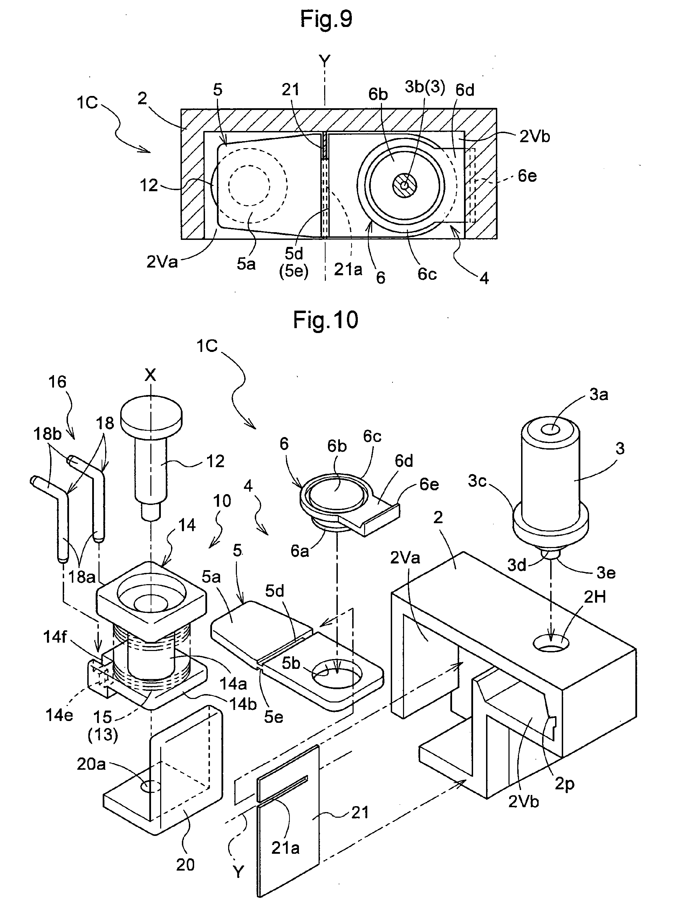

The solenoid unit 10 includes a fixed iron core 12 extending along an axis X, a solenoid coil 13 for selectively magnetizing the fixed iron core 12, and a yoke 20 for converging a magnetic field generated by the solenoid coil 13. The solenoid coil 13 includes a bobbin 14 disposed outwardly around the outer peripheral face of the fixed iron core 12, a wire 15 wound in the form of a coil about a cylindrical portion 14a of the bobbin 14, and an energizing portion 16 for energizing (power feeding to) the wire 15.
A valve portion 4 includes a pivotal member 5 (movable member) pivotally supported by a portion of the plate-like member 21 disposed erect laterally of the solenoid coil 13. The pivotal member 5 is supported to be pivotable via a pivot axis Y (see Fig. 9) formed within the plane of the plate-like member 21. At one end of the pivotal member 5, there is provided an attracted portion 5a which is attracted to an upper end of the fixed iron core 12 that is magnetized in response to energization to the solenoid coil 13. As the attracted portion 5a is attracted to the upper end of the fixed iron core 12, the pivotal member 5 assumes a first posture for closing the passageway 3a of the nozzle unit 3, as shown in Fig. 8 (a).

Adjacent the other end of the pivotal member 5 which end corresponds to the opposite end away from the attracted portion 5a across the pivot axis, a circular through hole 5b is formed. Within this through hole 5b, the valve body 6 formed of an elastic material such as rubber is retained. The elastic material forming the valve body 6 can be e.g. a synthetic rubber (e.g. NBR) having a shore hardness value of about 40. However, the material is not limited thereto, but any other material having appropriate elasticity for use as a valve body in the invention can be used also. The valve body 6 integrally includes a cylindrical retained portion 6a which is engaged and inserted from above the through hole 5b of the pivotal member 5, a flat disc-like upper face portion 6b that closes the upper end of the retained portion 6a, a flange portion 6c extending radially outward from the circumference of the upper end of the retained portion 6a, and an extension portion 6d (an example of "discharge operation portion") extending further radially to one side from a portion of the flange portion 6c.

Under the first posture where the attracted portion 5a is attracted to the upper end of the fixed iron core 12, as shown in Fig. 8 (a), the upper face portion 6b of the valve body 6 is pressed against an open end face 3e of the nozzle unit 3, whereby the passageway 3a of the nozzle unit 3 is closed. In this, the extension portion 6d of the valve body 6 is elastically deformed to be bent downward, by a projection-like, operation assisting portion 2p formed downward from the inner face of the housing 2. At the same time, the upper face portion 6b of the valve body 6 is also elastically deformed, with its center portion projecting downward. Incidentally, inside the passageway 3a of the nozzle unit 3, there is formed an orifice with an inner diameter of about 0.3 mm which is very small relative to the inlet opening formed at the upper end of the passageway 3a. As this orifice 3b extends continuously to the lower end of the passageway 3a against which the valve body 6 is pressed, reliable closure is made possible even with a relatively small pressing force for pressing the valve body 6 against the nozzle unit 3.

Upon release of energization (power supply) to the solenoid coil 13, this eliminates the magnetization of the fixed iron core 12 and the magnetic attraction of the attracted portion 5a. Hence, the pivotal member 5 is switched over to a second posture shown in Fig. 8 (b), due to elastic resilience of the extension portion 6d of the valve body 6 that urges this portion 6d to return to its shape extending straight along the upper face portion 6b, elastic resilience of the upper face portion 6b of the valve body 6 which urges this face portion 6b to return to its flat shape without any projection and also the pressure of air discharged from the cuff (in case compressed air is present in the cuff of the sphygmomanometer). Under this second posture, the condition of the valve body 6 being pressed against the open end portion 3c is released, so the passageway 3a is opened.

The extension portion 6d of the valve body 6 has a rectangular shape in its plane view and from the vicinity of its straight side at the leading end, there is upwardly extended a pawl-like projection 6e engageable with a sharp end of the operation assisting portion 2p. This pawl-like portion 6e, which has a triangular cross section and extends straight through the plane of Fig. 8, provides a function of causing the extension portion 6e to engage the operation assisting portion 2p in a reliable manner when the pivotal member 5 is under the first posture. Further, since the pawl-like projection 6e is configured to maintain the engaged posture engaged with the operation assisting portion 3p under the second posture already, there is no risk of being switched over to the first posture without engagement between the pawl-like projection 6e and the operation assisting portion 2p. The pawl-like projection 6e can be sized to have a height of 0.2 to 0.4 mm approximately. The value is not limited thereto, however.

As shown in Fig. 8 and Fig. 10, the plate-like member 21 supporting the pivotal member 5 is clamped and fixed between the yoke 20 and the housing 2 to extend substantially parallel to the axis X. Slightly downwardly of the upper end of the plate-like member 21, there is formed a slit-like cutout portion 21a intersecting the axis X at the right angle. As the cutout portion 21a is open only on one side of the plate-like member 21, the vicinity of this cutout portion 21a presents a one-side opened rectangular shape. In the front and back faces of the pivotal member 5 adjacent its center portion, there are formed a pair of straight retaining grooves 5d, 5e disposed in opposition to each other. The pivotal member 5 is inserted to the slit of the cutout portion 21a, with the cutout portion 21a being retained in the pair of retaining grooves 5d, 5e at one time. The retaining grooves 5d, 5e of the pivotal member 5 and the cutout portion 21a of the plate-like member 21 cooperate to constitute a supporting means for pivotally supporting the pivotal member 5 and to constitute also a restricting means for restricting movement and rotation (in particular, rotation about the axis X) of the pivot axis Y of the pivotal member 5.

The energizing portion 16 includes a pair of bar-like terminals 18 bent in the L-shape. Each bar-like terminal 18 is retained as being pressed into and engaged within a groove formed in a lower flange portion 14b projecting radially from the lower end of the bobbin 14. More particularly, the terminal 18 consists of a straight extending terminal body 18a and a connected portion 18b extending integrally at a right angle from the upper end of the terminal body 18a.
The lower flange portion 14b of the bobbin 14 defines a receiving hole 14e capable of receiving therein the terminal body 18a along the axis X and an engaging groove 14f communicated to the receiving hole 14e and extending radially. In operation, the terminal body 18a of the terminal 18 will be inserted from above the receiving hole 14e of the lower flange portion 14b and then the connected portion 18b of the terminal body 18 will be pressed into the engaging groove 14f. With these simple and easy operations, fixing of the terminal 18 to the bobbin 14 will be completed. The wire 15 constituting the solenoid coil 13 is directly welded to the leading end portion or intermediate portion of the connected portion 18b. With the engagement between the engaging groove 14f and the connected portion 18b, inadvertent withdrawal of the terminal 18 along the axis X and the pivotal displacement of the terminal 18 within the receiving hole 14e are restricted.

Next, an example of assembly operation of the solenoid valve 1C will be described next.
(1) The wire 15 is wound around the cylindrical portion 14a of the bobbin 14, thus forming the solenoid coil 13.
(2) The terminal bodies 18a of the terminals 18 are inserted from above into the receiving hole 14e of the lower flange portion 14b and the connected portions 18b of the terminals 18 are pressed into the engaging grooves 14f, whereby the pair of terminals 18 are fixed to the bobbin 14. The opposed terminal ends of the wire 15 are welded to the connected portions 18b.
(3) The fixed iron core 12 is inserted into the cylindrical portion 14a of the bobbin 14 to which the solenoid coil 13 is supported. After the lower end of the fixed iron core 12 is inserted into the through hole 20a of the yoke 20, this is fastened by caulking.
(4) The retained portion 6a of the valve body 6 is fitted into the through hole 5b of the pivotal member 5.
(5) The pivotal member 5 is inserted along the lateral direction to the cutout portion 21a of the plate-like member 21. In this, care will be taken such that the portions of the plate-like member 21 located to vertically clamp the slit of the cutout portion 21a may be inserted into the pair of retaining grooves 5d, 5e of the pivotal member 5.
(6) The assembly consisting of the pivotal member 5 and the plate-like member 21 is accommodated laterally within the cavity portion 2Va of the housing 2.
(7) The assembly of the solenoid coil 13 is press-fitted laterally into the cavity portion 2Va of the housing 2. In doing this, care is taken such that the plate-like member 21 accommodated there in advance may be clamped and fixed between the yoke 20 and the inner wall of the housing 2.
(8) The nozzle unit 3 is attached to the housing 2. Adjacent the lower end of the nozzle unit 3, there are formed a flange 3c which is generally circular in its plane view and a small-diameter boss portion 3d extending downward from the flange 3c. The boss portion 3d is pres-fitted into a through hole 2Hof the housing so that the lower end face of the flange 2c may be placed in gapless contact with the upper face of the housing 2.

### [Fourth Embodiment]

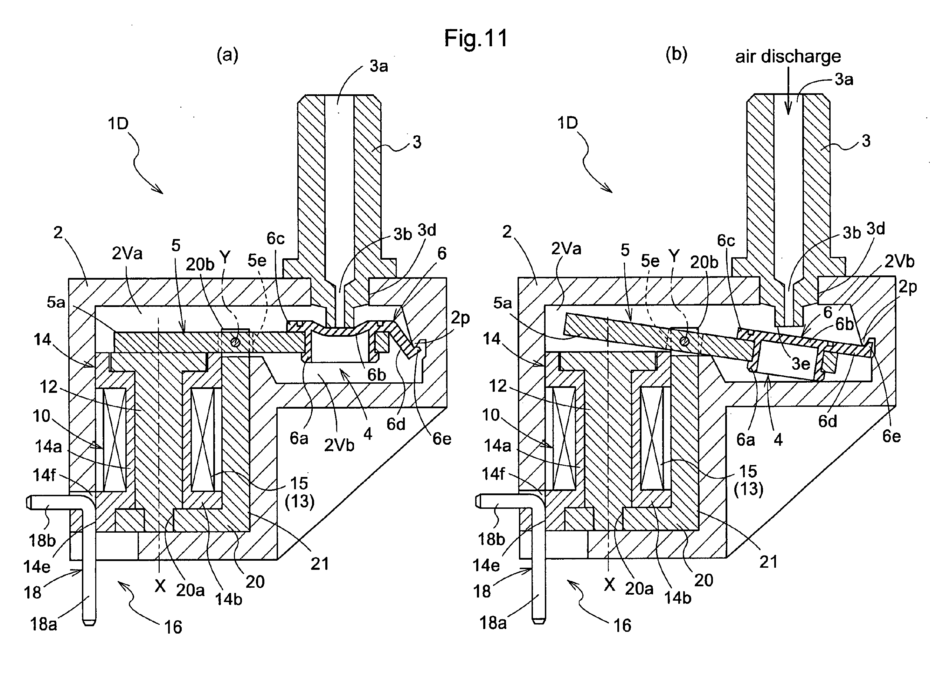

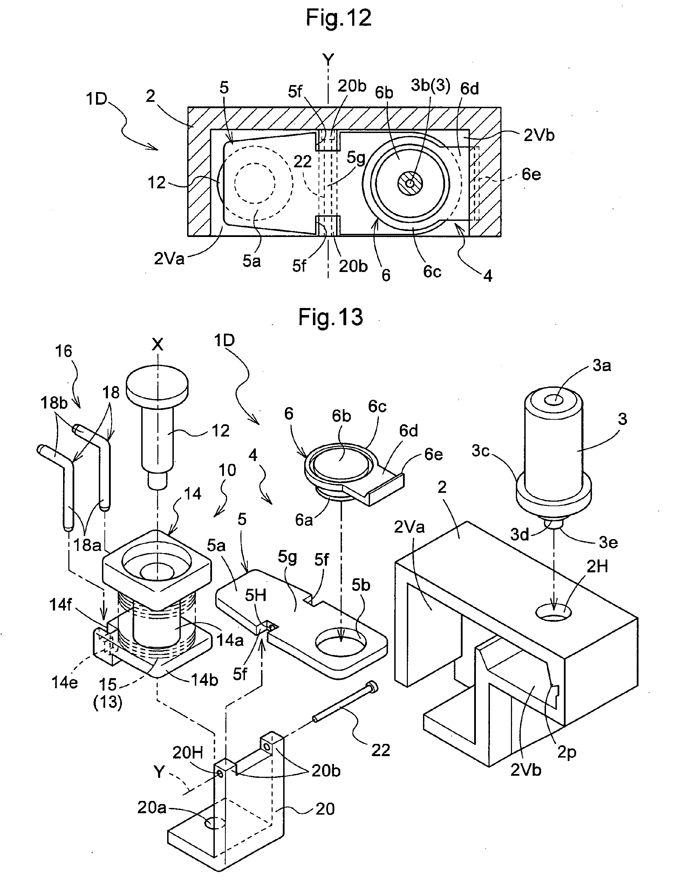

Figs. 11-13 show a solenoid valve 1D according to a fourth embodiment. In this solenoid valve 1D, the restricting means for restricting movement of the pivotal axis Y of the pivotal member 5 comprises a first retaining hole 5H formed in the pivotal member 5 to extend along the pivotal axis Y, a second retaining hole 20H formed at the leading end of the yoke 20, and a shaft member 22 inserted to the first retaining hole 5H and the second retaining hole 20H simultaneously.
In this case, adjacent the center portion of the pivotal member 5, there is formed a narrow portion 5g sandwiched between a pair of cutouts 5f. On the other hand, at opposed ends of the leading end of the yoke 20, there are formed a pair of extension portions 20b, 20b for receiving this narrow portion 5g therebetween, with the extension portions 20b, 20b extending upward. At the narrow portion 5g of the shaft member 22, the first retaining hole 5H extends horizontally therethrough. And, at the extension portions 20b, 20b of the yoke 20 too, the pair of horizontally extending second retaining holes 20H extend therethrough. As the shaft member 22 is inserted to and through these first retaining hole 5H and the second retaining holes 20H, the pivotal member 5 is pivotally supported to the yoke 20. The first retaining hole 5H of the pivotal member 5, the second retaining holes 20H of the yoke 20 and the shaft member 22 together constitute a restricting means for restricting movement and rotation of the pivotal axis Y during the pivotal operation of the pivotal member 5.

### (Modified Embodiments)

By further modifying the embodiment shown in Figs. 11-13, the pivotal member 5 may be pivotally supported by the following arrangement. Namely, a rectangular through hole is formed adjacent the center portion of the pivotal member 5. Whereas, adjacent the center of the leading end of the yoke 20, there is formed a projection which advances into this through hole of the pivotal member 5. Then, by inserting a single shaft member into both the lateral through hole formed at the projecting portion of the yoke 20 and the lateral through hole formed in the pivotal member 5, the pivotal member 5 is pivotally supported.

Instead of providing the separate urging means as provided in the first embodiment and the second embodiment, the vicinity of the center portion of the valve body made of elastic material and pressed against one terminal end of the nozzle unit under the first posture may be configured to act also as an urging means for switching over the pivotal member to the second posture, in accordance with release of energization to the solenoid coil.

### Industrial Applicability

As described above, the solenoid valve according to the present invention has the advantageous features of not requiring high precision working technique for the movable member, the disposing position of the nozzle unit being less restricted, the solenoid valve being readily made light-weight and compact, the stability of the valve operation, etc. The inventive solenoid valve may be suitably used in medical apparatuses. In particular, the inventive solenoid valve can be used as a discharge valve employed in a medical apparatus, e.g. an automated sphygmomanometer, for discharging air at a constant and very low speed. In particular, the invention may be employed in a finger sphygmomanometer, a wrist sphygmomanometer, an ear sphygmomanometer, having a small cuff capacity. Further, the invention may be suitably employed in a brachial sphygmomanometer (an integrated sphygmomanometer integrating a cuff and a body). Furthermore, the inventive valve can be suitably employed in any other medical apparatuses such as an air discharge valve in a massaging apparatus for ankles, a respiration synchronizer (demand valve) in an oxygen concentrator, an air discharge valve in an air cylinder forming a clamp mechanism of an automated peritoneal dialysis apparatus, etc.

## Claims

1. A solenoid valve comprising:
a fixed iron core extending along an axis;
a solenoid coil disposed around the fixed iron core;
a nozzle unit having a discharge passageway;
a valve body for closing the discharge passageway of the nozzle unit; and
a movable member supporting the valve body and movable, depending on an energization state to the solenoid coil, between a first posture for closing the discharge passageway with the valve body and a second posture for opening the discharge passageway;
**characterized in that**
the movable member comprises a pivotal member (5) supported to be pivotable via a pivot axis (Y) between the first posture and the second posture;
at one end of the pivotal member (5), there is provided an attracted portion (5a) configured to realize the first posture as this attracted portion is electromagnetically attracted to the fixed iron core (12) in accordance with energization to the solenoid coil (13); and
at the other end away from said one end across the pivot axis (Y), there is provided the valve body (6) for closing the discharge passageway (3a) in said first posture.

2. The solenoid valve according to claim 1, **characterized in that** there is provided an urging mechanism (6d,6e,2p;30) for urging the pivotal member (5) toward the second posture where the valve body (6) is removed from an opening (3e) of the discharge passageway (3a).

3. The solenoid valve according to claim 2, **characterized in that** the valve body (6) comprises an elastic member having a closing portion (6b) that is elastically pressed against the opening (3e) of the nozzle unit (3) under the first posture; and
the urging mechanism includes an operation assisting portion (2p) provided in a housing (2) which houses the pivotal member (5) therein and a discharge operation portion (6d) formed as a projecting portion of the valve body (6) so as to elastically urge the pivotal member (5) toward the second posture in response to engagement thereof with the operation assisting portion (2p).

4. The solenoid valve according to claim 3, **characterized in that** the discharge operation portion (6d) is configured to maintain its engagement with the operation assisting portion (2p) under both the first posture and the second posture.

5. The solenoid valve according to any one of claims 1-4, **characterized in that** there is provided a restricting means (2b;5d,5e,21;5H,20H,22) for restricting movement of a pivot axis (Y) included in the pivotal member (5).

6. The solenoid valve according to claim 5, **characterized in that** the restricting means includes a pair of retaining grooves (5d,5e) disposed in opposition to each other in front and back faces of the pivotal member (5) and a plate-like member (21) having a slit-like cutout (21a) that comes into engagement with the pair of retaining grooves simultaneously.

7. The solenoid valve according to claim 5, **characterized in that** a plate-like yoke (20) is disposed adjacent the solenoid coil (13); and
the restricting means includes a first retaining hole (5H) formed in the pivotal member (5) to extend along the pivot axis (Y), a second retaining hole (20H) formed at the leading end of the yoke (20), and a shaft member (22) inserted into the first retaining hole (5H) and the second retaining hole (20H) simultaneously.
